(19) Europäisches Patentamt
European Patent Office
Office européen des brevets

(11) **EP 1 891 985 B1**

(12) **EUROPÄISCHE PATENTSCHRIFT**

(45) Veröffentlichungstag und Bekanntmachung des Hinweises auf die Patenterteilung:
**06.03.2013   Patentblatt 2013/10**

(21) Anmeldenummer: 07013428.3

(22) Anmeldetag: **10.07.2007**

(51) Int Cl.:
*A61L 27/04* *(2006.01)*      *A61L 27/30* *(2006.01)*
*A61L 27/58* *(2006.01)*      *A61L 31/02* *(2006.01)*
*A61L 31/08* *(2006.01)*      *A61L 31/14* *(2006.01)*
*A61L 31/18* *(2006.01)*

(54) **Markerlegierung Mg(x)Yb(y)M(z)**

Marker alloy Mg(x)Yb(y)M(z)

Alliage de marqueur Mg(x)Yb(y)M(z)

(84) Benannte Vertragsstaaten:
**CH DE FR GB IE LI**

(30) Priorität: **07.08.2006   DE 102006038237**

(43) Veröffentlichungstag der Anmeldung:
**27.02.2008   Patentblatt 2008/09**

(73) Patentinhaber: **Biotronik VI Patent AG
6341 Baar (CH)**

(72) Erfinder:
• **Zberg, Bruno
6460 Altdorf (CH)**
• **Gerold, Bodo, Dr.
91225 Zellingen (DE)**
• **Löffler, Jörg, Prof., Dr.
8049 Zürich (CH)**

(74) Vertreter: **Lindner-Vogt, Karin L.
Biotronik SE & Co. KG
Woermannkehre 1
12359 Berlin (DE)**

(56) Entgegenhaltungen:
**EP-A- 1 632 256     DE-A1- 10 361 942**

• **HEHMANN F ET AL: "Extension of solid solubility in magnesium by rapid solidification" MATERIALS SCIENCE & ENGINEERING A (STRUCTURAL MATERIALS: PROPERTIES, MICROSTRUCTURE AND PROCESSING) SWITZERLAND, Bd. A125, Nr. 2, 1. Juni 1990 (1990-06-01), Seiten 249-265, XP002465542 ISSN: 0921-5093**

**Beschreibung**

Die Erfindung betrifft eine neue Markerlegierung für ein Implantat aus einem biodegradierbaren metallischen Werkstoff sowie deren Verwendung in diesem Zusammenhang.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube. Häufig ist nur eine temporäre Stütz- beziehungsweise Haltefunktion bis zum Abschluss des Heilungsprozesses oder Stabilisierung des Gewebes notwendig beziehungsweise erwünscht. Um Komplikationen zu vermeiden, die aus dem dauerhaften Verbleib der Implantate im Körper resultieren, müssen die Implantate entweder wieder operativ entfernt werden oder sie bestehen aus einem Werkstoff, der allmählich im Körper abgebaut wird, das heißt biokorrodierbar ist. Die Zahl biokorrodierbarer Werkstoffe auf der Basis von Polymeren oder Legierungen ist stetig gewachsen. So sind unter anderen biokorrodierbaren Metalllegierungen der Elemente Magnesium, Eisen und Wolfram bekannt.

EP 1 270 023 beschreibt eine Magnesiumlegierung, die sich für endovaskuläre und orthopädische Implantate eignet. Die Legierung kann bis zu 5 Gewichtsprozent seltene Erden enthalten. Die aus dem Stand der Technik bekannten biokorrodierbaren Metalllegierungen und Polymere für medizinische Implantate haben jedoch den Nachteil, dass sie nicht oder nur in einem nicht zufrieden stellenden Ausmaß im gängigen Röntgenverfahren sichtbar sind. Zur postoperativen Überwachung des Heilungsfortschrittes oder zur Kontrolle minimalinvasiver Eingriffe ist aber gerade die Röntgendiagnostik ein wichtiges Instrumentarium. So werden beispielsweise seit einigen Jahren Stents in den Koronararterien bei der akuten Myokardinfarkttherapie platziert. Nach gängigen Verfahren wird ein Katheter, der den Stent in einem nicht expandierten Zustand trägt, im Bereich der Läsion der koronaren Gefäßwand positioniert. Anschließend weitet sich der Stent entweder durch selbstexpansive Kräfte oder durch Inflation eines Ballons auf, um im expandierten Zustand eine Obstruktion der Gefäßwand zu verhindern. Der Vorgang der Positionierung und Expansion der Stents muss während der Prozedur durch den Kardiologen laufend überwacht werden.

Im medizinischen Bereich wird mit Röntgenstrahlen im Energiebereich von 60 bis 120 keV, bei Anwendung am Herz typischer Weise im Bereich von 80 bis 100 keV gearbeitet. Da der Röntgenabsorptionskoeffizient stark von der Energie abhängt, ist dieser Arbeitsbereich bei der Auswahl von geeigneten Markermaterialien zu berücksichtigen. Die Absorption (Intensitätsabschwächung) der Röntgenstrahlen kann vereinfacht mit einem exponentiellen Abschwächungsgesetz beschriben werden.

$$\frac{I}{I_0} = \exp\left[-\left(\frac{\mu}{\rho}\right)x\right]$$

Dabei ist I die gemessenen Intensität nach dem Probendurchgang, $I_0$ die Intensität der Strahlung vor dem Probendurchgang, $\mu/\rho$ der Massenabsorptionskoeffizient und x die Massendicke der Probe. x berechnet sich aus der Dicke t mal der Dichte des Stoffes $\rho$, $x = \rho \cdot t$. Für Legierungen berechnet sich der Massenabsorptionskoeffizient durch Aufsummieren der Komponenten.

Bei geringer Absorption des gewählten Werkstoffs in einem gegebenen Energiebereich der Röntgenabsorption könnte demnach durch Erhöhung der Materialdicke eine Verbesserung der Röntgensichtbarkeit erreicht werden; jedoch stößt diese Maßnahme schnell an ihre Grenzen, insbesondere, wenn es um die Markierung filigraner Strukturen, wie sie bei Stents vorliegen, geht.

Es ist somit bekannt, Implantate mit einem Marker in Form einer Beschichtung, eines Bandes, eines Inlays oder andersartig angeformten Design zur Verbesserung der Röntgensichtbarkeit auszustatten. Beispielsweise werden Metallbänder aus Gold oder anderen Edelmetallen in bestimmten Bereichen eines Stents angebracht.

DE 103 61 942 A1 beschreibt einen radioopaken Marker für medizinische Implantate, der 10 bis 90 Gewichtsprozente einer biokorrodierbaren Basiskomponente, insbesondere aus der Gruppe der Elemente Magnesium, Eisen und Zink, enthält. Weiterhin enthält der Marker 10 bis 90 Gewichtsprozente eines oder mehrerer radioopaker Elemente aus der Gruppe I, Au, Ta, Y, Nb, Mo, Ru, Rh, Ba, La, Ce, Pr, Nd, Sm, Eu, Gd, Tb, Dy, Ho, Er, Tm, Yb, Lu, Hf, Ta, W, Re, Os und Bi als Markerkomponente. Die beschriebenen Marker eignen sich demnach prinzipiell für den Einsatz in biokorrodierbaren Implantaten, insbesondere solchen aus biokorrodierbaren Magnesiumlegierungen.

Bei Implantaten aus biokorrodierbaren metallischen Werkstoffen auf der Basis von Magnesium, Eisen oder Wolfram bestehen zumeist weitere Anforderungen an das Markermaterial:

- der Marker sollte durch die korrosiven Prozesse nicht frühzeitig vom Grundkörper des Implantates getrennt werden, um einer Fragmentbildung und damit der Gefahr der Embolisation vorzubeugen;

- der Marker sollte nicht schneller als der Grundkörper degradieren, um in Nachuntersuchungen noch sichtbar zu bleiben; jedoch sollte eine zumindest teilweise in vivo Degradation gegeben sein;

- der Marker sollte schon bei geringen Materialdicken eine hinreichende Röntgendichte besitzen; und

- das Markermaterial sollte möglichst keinen oder allenfalls einen geringen Einfluss auf die Degradation des Grundkörpers haben.

Bei Einsatz von Markern aus metallischen Werkstoffen auf biokorrodierbaren metallischen Grundkörpern besteht jedoch das besondere Problem, dass aufgrund von elektrochemischen Wechselwirkungen zwischen den beiden Materialien die Degradation des Grundkörpers in einem Kontaktbereich zwischen Marker und Grundkörper verändert, d. h. in der Regel beschleunigt ist. Ferner ist eine Verarbeitung des Markermaterials aufgrund der häufig geringeren Schmelzpunkte des Grundmaterials erschwert; Bearbeitungsverfahren, wie Löten oder Laserschweißen, oder auch das Eintauchen des Implantates in eine Schmelze aus dem Markerwerkstoff sind zumeist nicht möglich.

Der vorliegenden Erfindung lag daher die Aufgabe zugrunde, eine auf die vorgenannten Bedürfnisse spezifisch zugeschnittene Markerlegierung bereitzustellen, mit der die geschilderten Nachteile des Standes der Technik überwunden werden können.

Diese Aufgabe wird durch die erfindungsgemäße Markerlegierung für ein Implantat aus einem biokorrodierbaren metallischen Werkstoff und der Zusammensetzung (1)

$$Mg_xYb_yM_z \qquad (1)$$

mit

x = 10 - 60 Atomprozent;

y = 40 - 90 Atomprozent;

z = 3 - 8 Atomprozent;

M einem oder mehreren Elementen ausgewählt aus der Gruppe Ag, Zn, Au, Ga, Pd, Pt, Al, Sn, Ca, Nd, Ba, Si und Ge; sowie

unter der Maßgabe, dass x, y und z zusammen und einschließlich herstellungsbedingter Verunreinigungen 100 Atomprozent ergeben,

gelöst. Die erfindungsgemäße Markerlegierung zeichnet sich (i) durch ihren geringen Schmelzpunkt (dieser liegt bei den angegebenen Legierungszusammensetzungen bei etwa 450°C bis 800°C) und damit besondere Eignung für herkömmliche thermische Bearbeitungsverfahren, wie Löten oder Laserschweißen, (ii) ein homogenes Gefüge ohne intermetallische Phasen, was die Verarbeitbarkeit vereinfacht, und (iii) eine (zumindest teilweise) Biokorrodierbarkeit aus. Durch eine geeignete Wahl der Herstellungsparameter lassen sich sowohl ein homogenes Gefüge (Mischkristall) als auch die Entstehung von intermetallischen Phasen steuern. Diese Herstellungsparameter beinhalten im wesentliche die Zusammensetzung der Schmelze selbst, die Temperatur der Marker-Schmelze und des Substrats, die Umgebungsatmosphäre (inert z.B. Vakuum oder Argongas; reaktiv z.B. Stickstoff) und deren Druck sowie die Abkühlrate und weiter anschließende Wärmebehandlungsmaßnahmen, die wiederum im wesentlichen durch die Temperatur sowie Aufheiz- und Abkühlrate und die Umgebungsatmosphäre gekennzeichnet sind.

Vorzugsweise ist x = 25 bis 40 Atomprozent und y = 60 bis 75 Atomprozent. Es hat sich gezeigt, dass Markerlegierungen der genannten Zusammensetzungen einen für den medizintechnischen Röntgenenergiebereich von 80 keV bis 100 keV hinreichend hohen mittleren Massenabsorptionskoeffizient besitzen als auch eine Schmelztemperatur aufweisen, die unter den Schmelzpunkten der bisher eingesetzten biokorrodierbaren Magnesiumlegierungen für die Grundkörper der Implantate liegt. Weiterhin sind Markerlegierungen der genannten Zusammensetzung auch in wässriger oder physiologischer Lösung für die angestrebten Zwecke hinreichend lange stabil.

Der Zusatz der Komponente M dient insbesondere der Erniedrigung des Schmelzpunktes der Legierung und ist z = 3 bis 8 Atomprozent.

Die Legierungszusammensetzung $Mg_{31,5}Yb_{68,5}$ ist ein Eutektikum, dessen Schmelzpunkt bei etwa 496°C liegt, während beispielsweise der biokorrodierbare Magnesiumwerkstoff WE43 einen Schmelzpunkt von etwa 590°C aufweist. Aus der Dichte dieser Markerlegierung (5,9 g/cm$^3$) und deren mittlerer Massenabsorptionskoeffizient im Energiebereich von 80 bis 100 keV (5,98 cm$^2$/g) lässt sich eine benötigte Materialdicke für eine Abschwächung der Intensität auf den Faktor 0,86 von 51 μm errechnen. Dieser Wert ist deutlich geringer als die Wanddicke üblicher Magnesiumstents. Der genannte Faktor entspricht einem Abschwächungskoeffizienten, wie er bei goldbeschichteten Stahlstents zu beobachten ist, wobei eine Dicke des Stahls bei 70 μm und einer Dicke der Goldbeschichtung bei 14 μm liegt. Mit anderen Worten, es wurde eine Materialdicke der Markerlegierung berechnet, die notwendig ist, um dieselbe Intensitätsabschwächung wie beim Stahl/Gold-Verbund zu erhalten und der ermittelte Wert von 51 μm verdeutlicht, dass diese Markerlegierung für die filigranen Strukturen von Stents geeignet ist.

Ein besonderer Vorteil der erfindungsgemäßen Markerlegierung liegt darin, dass die E-lektronegativität von Ytterbium geringer ist als die von Magnesium, so dass eine Beschleunigung der Korrosion des Grundkörpers im Kontaktbereich zum Markermaterial durch Bildung von Lokalelementen verhindert wird.

Vorzugsweise ist der biodegradierbare Werkstoff des Implantats eine Legierung eines Elements ausgewählt aus der Gruppe Magnesium, Eisen und Wolfram, insbesondere eine biokorrodierbare Magnesiumlegierung, wie WE43. Die genannten Elemente liegen dabei in der Legierung als Hauptkomponente vor, d.h. ihr Massenanteil ist im Vergleich zu den weiteren in der Legierung vorhanden Elementen am größten. Vorzugsweise liegt der Massenanteil der genannten Elemente an den biokorrodierbaren Legierungen bei mehr als 50 Gew.%, insbesondere mehr als 70 Gew.%.

Besonders bevorzugt wird als Implantatswerkstoff eine biokorrodierbare Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Die biokorrodierbaren Legierungen der Elemente Magnesium, Eisen oder Wolfram für den Grundkörper des Implantats sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, $CaCl_2$ 0,2 g/l, KCl 0,4 g/l, $MgSO_4$ 0,1 g/l, $NaHCO_3$ 2,2 g/l, $Na_2HPO_4$ 0,126 g/l, $NaH_2PO_4$ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Röntgenmarker liegt vorzugsweise in massiver Ausführung als Vollmaterial vor. Alternativ kann der Röntgenmarker jedoch auch als Pulver in eine organische Trägermatrix eingebettet sein.

Vorzugsweise ist das Implantat ein Stent, insbesondere aus einer Magnesium- oder Eisenlegierung (z. B. der Magnesiumlegierung WE43). Hier besteht ein erheblicher Bedarf nach Markermaterialien, die sich aus den besonderen Anforderungen an das Design und den Werkstoff des Stents ergeben.

In einer besonderen Ausführungsform ist das Implantat aus der Markerlegierung hergestellt.

Die Legierung wurde hergestellt durch gemeinsames Aufschmelzen der Legierungskomponenten in einem Graphit bzw. Bornitridtiegel, konkret durch gemeinsames Aufschmelzen von 31.5 Atomprozent Magnesium und 68.5 Atomprozent Ytterbium. Da sowohl Magnesium als auch Ytterbium beide sehr hohe Oxidationsneigung und tiefe Verdampfungsenthalpien besitzen, wurde der Schmelzprozess unter Schutzgas und bei leichtem Überdruck durchgeführt.

Ein Stent aus der Magnesiumlegierung WE43 (enthält 93 Gew.% Magnesium, 4 Gew.% Yttrium (W) und 3 Gew.% Seltenerdmetalle außer Yttrium (E)) wird beidseitig mit seinen Enden bis zu einer Tiefe von ca. 1 mm und für 1 - 2 s in eine Schmelze aus $Mg_{31.5}Yb_{88.5}$ getaucht und anschließend abgekühlt. Die abgekühlte Schicht aus dem Markermaterial ist ca. 50 μm dick.

## Patentansprüche

1. Markerlegierung für ein Implantat aus einem biodegradierbaren metallischen Werkstoff und mit der Zusammensetzung (1)

$$Mg_xYb_yM_z \quad (1)$$

mit
x = 10 - 60 Atomprozent;

y = 40 - 90 Atomprozent;

z = 3 - 8 Atomprozent;

M einem oder mehreren Elementen ausgewählt aus der Gruppe Ag, Zn, Au, Ga, Pd, Pt, Al, Sn, Ca, Nd, Ba, Si und Ge; sowie

unter der Maßgabe, dass x, y und z zusammen und einschließlich herstellungsbedingter Verunreinigungen 100 Atomprozent ergeben.

**2.** Markerlegierung nach Anspruch 1, bei der
x = 25 - 40 Atomprozent;
y = 60 - 75 Atomprozent; und
z = 3 - 8 Atomprozent
ist.

**3.** Markerlegierung nach einem der vorhergehenden Ansprüche, bei der der biodegradierbare metallische Werkstoff des Implantats eine Legierung eines Elementes ausgewählt aus der Gruppe Magnesium, Eisen und Wolfram ist.

**4.** Verwendung einer Markerlegierung gemäß einem der Ansprüche 1 bis 3 für einen Röntgenmarker für ein Implantat aus einer biodegradierbaren Magnesiumlegierung.

**5.** Markerlegierung nach einem der vorhergehenden Ansprüche 1 bis 3, bei der das Implantat ein Stent ist.

**6.** Implantat bestehend aus einer Markerlegierung nach einem der Ansprüche 1 bis 3.

## Claims

**1.** A marker alloy for an implant made of a biodegradable metallic material with the composition (1)

$$Mg_xYb_yM_z \quad (1)$$

where
x = 10 to 60 atom percent;
y = 40 to 90 atom percent;
z = 3 to 8 atom percent; and
M is one or more elements selected from the group consisting of Ag, Zn, Au, Ga, Pd, Pt, Al, Sn, Ca, Nd, Ba, Si and Ge; and
with the proviso that x, y and z together, and including production-related impurities, make up 100 atom percent.

**2.** The marker alloy according to claim 1, wherein
x = 25 to 40 atom percent;
y = 60 to 75 atom percent; and
z = 3 to 8 atom percent.

**3.** A marker alloy according to any one of the preceding claims, wherein the biodegradable metallic material of the implant is an alloy of an element selected from the group consisting of magnesium, iron and tungsten.

**4.** Use of a marker alloy according to any one of claims 1 to 3 for an x-ray marker for an implant made of a biodegradable magnesium alloy.

**5.** A marker alloy according to any one of the preceding claims 1 to 3, wherein the implant is a stent.

**6.** An implant consisting of a marker alloy according to any one of claims 1 to 3.

## Revendications

**1.** Alliage de marqueur pour un implant, réalisé à partir d'un matériau métallique biodégradable, selon la composition (1) suivante :

$$Mg_xYb_yM_z \quad (1)$$

avec

x = 10 à 60 pour cent en atomes ;
y = 40 à 90 pour cent en atomes ;
z = 3 à 8 pour cent en atomes ;
M correspondant à un ou plusieurs éléments sélectionnés parmi le groupe Ag, Zn, Au, Ga, Pd, Pt, Al, Sn, Ca, Nd, Ba, Si et Ge ; et
à condition que x, y et z donnent conjointement un pourcentage atomique de 100, y compris les impuretés dues à la fabrication.

2.  Alliage de marqueur selon la revendication 1, dans lequel
    x = 25 à 40 pour cent en atomes ;
    y = 60 à 75 pour cent en atomes ; et
    z = 3 à 8 pour cent en atomes.

3.  Alliage de marqueur selon l'une des revendications précédentes, dans lequel le matériau métallique biodégradable de l'implant est un alliage d'un élément sélectionné parmi le groupe comprenant le magnésium, le fer et le tungstène.

4.  Utilisation d'un alliage de marqueur selon l'une des revendications 1 à 3, pour un marqueur de radiographie destiné à un implant réalisé à partir d'un alliage de magnésium biodégradable.

5.  Alliage de marqueur selon l'une des revendications précédentes 1 à 3, dans lequel l'implant est un stent.

6.  Implant constitué d'un alliage de magnésium selon l'une des revendications 1 à 3.

**IN DER BESCHREIBUNG AUFGEFÜHRTE DOKUMENTE**

**In der Beschreibung aufgeführte Patentdokumente**

- EP 1270023 A
- DE 10361942 A1